(19) Europäisches Patentamt
European Patent Office
Office européen des brevets

(11) **EP 1 967 840 A2**

(12) **EUROPEAN PATENT APPLICATION**

(43) Date of publication:
**10.09.2008 Bulletin 2008/37**

(51) Int Cl.:
*G01N 15/12* (2006.01)    *G01N 15/14* (2006.01)
*G01N 33/49* (2006.01)

(21) Application number: **08003550.4**

(22) Date of filing: **27.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **09.03.2007 JP 2007061093**

(71) Applicant: **SYSMEX CORPORATION**
**Kobe-shi,**
**Hyogo 651-0073 (JP)**

(72) Inventor: **Linssen, Jo**
**Kobe-shi**
**Hyogo 651-0073 (JP)**

(74) Representative: **HOFFMANN EITLE**
**Patent- und Rechtsanwälte**
**Arabellastrasse 4**
**81925 München (DE)**

(54) **Hematology analyzer, hematology analyzing method, and computer program product**

(57)    A hematology analyzer, obtains red blood cell scattered light information which is scattered light information related to red blood cells contained in a blood sample and reticulocyte scattered light information which is scattered light information related to reticulocytes contained in the blood sample; obtains a value equivalent to the amount of hemoglobin in the red blood cells from the red blood cell scattered light information; obtains a value equivalent to the amount of hemoglobin in the reticulocytes from the reticulocyte scattered light information; obtains difference between the hemoglobin amounts which is the difference between the value equivalent to the amount of hemoglobin in the red blood cells and the value equivalent to the amount of hemoglobin in the reticulocytes; and obtains information supporting clinical examination based on the value equivalent to the amount of hemoglobin in the reticulocytes and the difference between the hemoglobin amounts, is disclosed. A hematology analyzing method and a computer program product are also disclosed.

FIG.1

**Description**

FIELD OF THE INVENTION

The present invention relates to a hematology analyzer for analyzing hemocytes in a blood sample, hematology analyzing method, and a computer program product used in same.

BACKGROUND

Examples of anemia include iron deficiency anemia (IDA), anemia of chronic disorder (ACD), and megaloblastic anemia (MA) caused by vitamin 12 or folic acid. For example, administration of iron supplement is extremely effective as a treatment for iron deficiency anemia, but not for ACD. The cause of the anemia is therefore important to the treatment of the anemia.

Although examinations for serum iron, serum ferritin, soluble transferrin receptor (sTfR) and the like are performed to manage changes of the iron in the blood (iron dynamics), these examinations present a problem regarding expense.

Administration of recombinant human erythropoietin (rHuEPO) is a widely used treatment for renal anemia. Renal anemia is a disease caused by reduced production and secretion of erythropoietin by the kidney. There are cases treating renal anemia in which not improvement has been obtained despite administration of rHuEPO. The causes for this are various and include functional iron deficiency as a result of administering rHuEPO. The administration of iron supplement in addition to rHuEPO is required in such cases.

Vitamin 12 and folic acid deficiency can obstruct the synthesis of nucleic acid. Red blood cells are rich in folic acid and the most readily influenced, which may lead to megaloblastic anemia.

Since anemia arises from various causes, ascertaining the efficacy of treatment at an early stage is essential. Therefore, attention has been given to methods for checking the occurrence and attributes of reticulocytes. Since reticulocytes are immature red blood cells, monitoring reticulocytes is regarded as promising for treatment of anemia when used an indicator to the status of future hemoglobin production.

For example, U.S. Application No. 2005-219527 discloses a method for calculating the average forward scattered light intensity of mature red blood cells (RBC-Y) and the average forward scattered light intensity of a reticulocyte population (RET-Y) specified in a two-dimensional distribution, calculating the average amount of hemoglobin (RET-He) contained in the reticulocytes based on RET-Y, calculating the average amount of hemoglobin (REC-He) contained in the mature red blood cells based on RBC-Y), and calculating the difference between RET-He and RBC-He (that is, Delta-He).

Although Japanese Laid-Open Patent Publication No. 2005-257450 describes parameters such as average amount of hemoglobin in reticulocytes, average amount of hemoglobin in mature red blood cells, and the difference between the average amounts of hemoglobin of the reticulocytes and mature red blood cells, the relationship between these parameters and any practical clinical use is not disclosed.

SUMMARY OF THE INVENTION

The scope of the present invention is defined solely by the appended claims, and is not affected to any degree by the statements within this summary.

A first aspect of the present invention is a hematology analyzer comprising: a detecting unit for irradiating a blood sample with light and obtaining optical information which comprises at least scattered light information from each cell contained in the blood sample; a cell classifying means for classifying red blood cells and reticulocytes among the cells based on the obtained optical information; a scattered light information obtaining means for obtaining red blood cell scattered light information which is scattered light information related to red blood cells, and reticulocyte scattered light information which is scattered light information related to reticulocytes based on the classification results of the cell classifying means; a hemoglobin amount obtaining means for obtaining a value equivalent to the amount of hemoglobin in the red blood cells from the red blood cell scattered light information, a value equivalent to the amount of hemoglobin in the reticulocytes from the reticulocyte scattered light information, and difference between the hemoglobin amounts which is the difference between the value equivalent to the amount of hemoglobin in the red blood cells and the value equivalent to the amount of hemoglobin in the reticulocytes; and a supporting information obtaining means for obtaining information supporting clinical examination based on the difference between the hemoglobin amounts and the value equivalent to the amount of hemoglobin in reticulocytes.

A second aspect of the present invention is a hematology analyzing method, comprising steps of: obtaining red blood cell scattered light information which is scattered light information related to red blood cells contained in a blood sample, and reticulocyte scattered light information which is scattered light information related to reticulocytes contained in the blood sample; obtaining a value equivalent to the amount of hemoglobin in the red blood cells from the red blood cell scattered light information; obtaining a value equivalent to the amount of hemoglobin in the reticulocytes from the reticulocyte scattered light information; obtaining difference between the hemoglobin amounts which is the difference between the value equivalent to the amount of hemoglobin in the red blood cells and the value equivalent to the amount of hemoglobin in the reticulocytes; and obtaining information supporting clinical examination based on the value equivalent to the amount of hemoglobin in the reticulocytes and the difference between the hemoglobin amounts.

A third aspect of the present invention is a computer program product, comprising: a computer readable medium; and instructions, on the computer readable medium, adapted to enable a general purpose computer to perform operations, comprising : a step of obtaining red blood cell scattered light information which is scattered light information related to red blood cells contained in a blood sample, and reticulocyte scattered light information which is scattered light information related to reticulocytes contained in the blood sample; a step of obtaining a value equivalent to the amount of hemoglobin in the red blood cells from the red blood cell scattered light information; a step of obtaining a value equivalent to the amount of hemoglobin in the reticulocytes from the reticulocyte scattered light information; a step of obtaining difference between the hemoglobin amounts which is the difference between the value equivalent to the amount of hemoglobin in the red blood cells and the value equivalent to the amount of hemoglobin in the reticulocytes; and a step of obtaining information supporting clinical examination based on the value equivalent to the amount of hemoglobin in the reticulocytes, and the difference between the hemoglobin amounts.

BRIEF DESCRIPTION OF THE DRAWINGS

FIG. 1 is a block diagram showing the entirety of an embodiment of the hematology analyzer of the present invention;
FIG. 2 is a structural view of the essential part of the fluid mechanism;
FIG. 3 is a perspective view of an optical detection unit;
FIG. 4 is a flow chart of the analysis process which provides status identifying information;
FIG. 5 is a distribution chart which includes mature red blood cells and reticulocytes as objects for analysis;
FIG. 6 is a correlation diagram representing the regions corresponding to identification codes;
FIG. 7 shows a display screen which displays the analysis results;
FIG. 8 is a flow chart of another analysis process which provides status identifying information; and
FIG. 9 is a correlation diagram in which analysis data are plotted in time series.

DETAILED DESCRIPTION OF THE PREFERRED EMBODIMENTS

The preferred embodiments of the present invention will be described hereinafter with reference to the drawings.

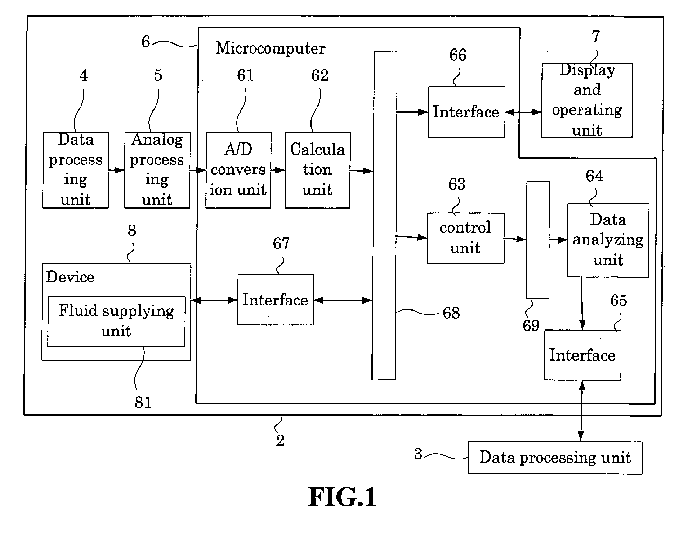

FIG. 1 is a block diagram showing the overall hematology analyzer. The hematology analyzer is configured by a measuring device 2 which has the functions of measuring aspirated blood and performing data processing and analysis, and a data processing unit 3 which has the function of analyzing and storing the data obtained by the measuring device 2. The data processing unit 3 is configured by software for hemocyte analysis installed on a commercial personal computer provided with a display screen, keyboard, hard disk and the like. The measuring device 2 is provided with a detection unit 4 for detecting hemocytes, an analog processing unit 5 for processing signals from the detection unit 4, a microcomputer 6 for processing the signals which have been processed by the analog processing unit 5, a display and operating unit 7 for inputting settings and the like and outputting results and the like, and a device 8 for driving the mechanisms and the like. The device 8 includes a fluid supplying device 81 for driving fluid elements such as valves, pumps and the like. Although the measuring device and data processing unit 3 are separate devices in the present embodiment, these devices may be integrated in a single device.

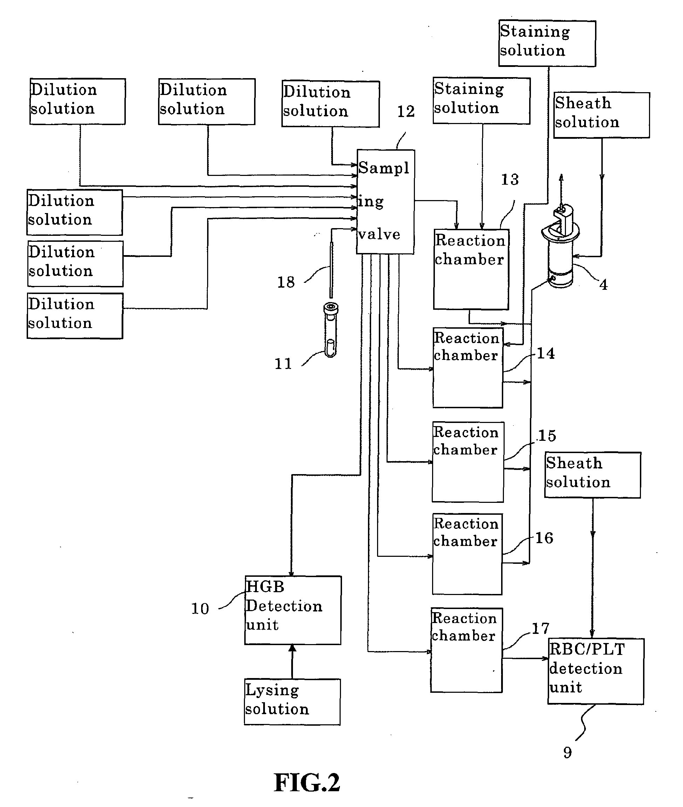

FIG. 2 is a block diagram showing the structure of the essential part of the fluid supplying unit 81. The fluid supplying unit 81 is provided with a sample aspirating nozzle 18, a sampling valve 12, and a plurality of reaction chambers 13 through 17. A blood sample accommodated in a sample container 11 is aspirated by the sample aspirating nozzle 18 and conducted to the sampling valve 12. The blood sample conducted to the sampling valve 12 is then divided in to measured doses for each item to be measured within the sampling valve 12, and the divided samples are then transferred to the reaction chamber 13 through 17 together with each reagent (dilution solution) supplied in fixed doses to the sampling valve 12. The dilution solution in this includes the meaning of a fluid having a hemolytic action and a fluid which does not have hemolytic action. A predetermined amount of staining solution is supplied to he reaction chamber according to the item of measurement, and a mixing and staining reaction occurs in the reaction chamber. A sample for measuring four types of white blood cells, a sample for measuring nucleated red blood cells, a sample for measuring reticulocytes, a sample for measuring white blood cells and basophils, and a sample for measuring red blood cells and platelets are respectively prepared in reaction chambers 13, 14, 15, 16, and 17. The prepared sample for measuring red blood cells and platelets is introduced to a detecting unit 9 of an electrical resistance measuring type, and particle measurement is performed. The prepared measurement samples for measuring four types of white blood cells, reticulocytes, white blood cells and basophils, are sequentially introduced to an optical type detecting unit 4, and sequential particle measurements are performed. Regarding hemoglobin, a measurement sample is transferred to the hemoglobin detection unit 10 together with a predetermined amount of dilution solution, a hemolytic agent is also supplied, and colorimetric measurement is performed by the detection unit 10. The hemoglobin measured by the detection unit 10 is the amount of hemoglobin contained in a predetermined volume of blood.

In the embodiment of the present invention, information which is practically useful for clinical examinations is obtained using the reticulocyte measurement sample. RET SEARCH II, a product supplied by Sysmex Corporation, may be used as the reagent mentioned above which is used for reticulocyte measurements. This reagent is available in a reagent kit which includes dilution solutions and stains; red blood cells, reticulocytes, and platelets are respectively stained by mixing and reacting 900 μl of dilution solution 9 and 18 μl of staining solution in 4.5 μl of blood.

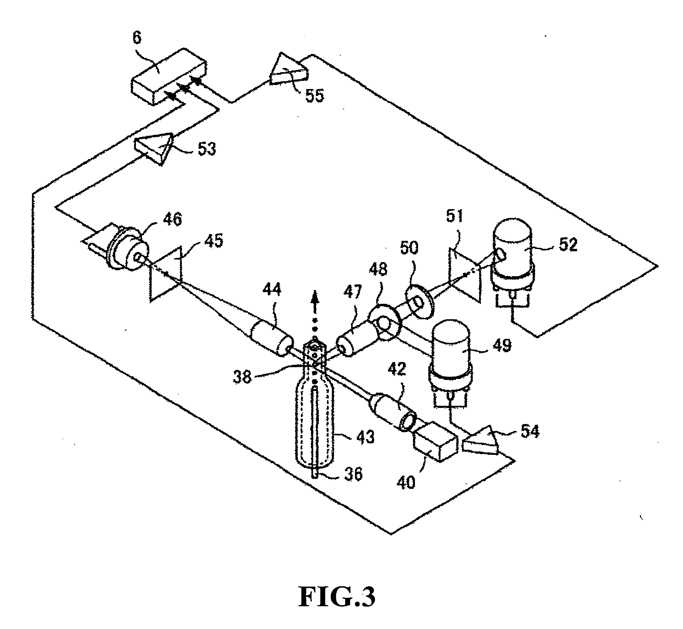

FIG. 3 is a perspective view of the optical detecting unit 4 provided in the hematology analyzer. The reticulocyte measurement sample prepared in the reaction chamber 15 is extruded at a constant speed through a nozzle 36 provided on the flow cell 43, and encapsulated by the surrounding sheath fluid to form a sheath flow which flows through an orifice 38 of the flow cell 43. A laser beam emitted from a laser diode 40 irradiates the orifice 38 of the flow cell 43 through a collimator lens 42. The forward scattered light, which is emitted from the blood cells that pass through the region irradiated by the laser beam at the orifice 38, impinges a photodiode 46 through a collective lens 44 provided with a bream splitter, and a pinhole plate 45. The side scattered light, which is emitted from the blood cells that pass through the region irradiated by the laser beam at the orifice 38, is collected by a collective lens 47. The side scattered light enters a photomultiplier tube (hereinafter referred to as "photomultiplier") 49 through a dichroic mirror 48, the side fluorescent light a photomultiplier 52 through the dichroic mirror 48, optical filter 50, and pinhole plate 51.

The forward scatter light signal impinging the photomultiplier 46, the side scattered light signal impinging the photomultiplier 4,and the side fluorescent light signal impinging the photomultiplier 52 are detected and photoelectrically converted by the detection units 53, 54, and 55, respectively, and subjected to various types of data processing by the microcomputer 6.

Similar signal detection and data analysis are performed for the other measurement samples in addition to the reticulocyte measurement sample.

As shown in FIG. 1, the microcomputer 6 is provided with an A/D conversion unit 61 for converting the analog signal from the analog processing unit 5 to digital signals; the A/D conversion unit 61 prepares the distribution data (2-parameter distribution data used as the basis for preparing a scattergram, and 1-parameter distribution data used as the basis for preparing a histogram) collected in the calculation unit 62.In the present embodiment, distribution data are prepared for the reticulocyte measurement sample using the forward scattered light intensity and the side fluorescent light intensity as parameters.

The microcomputer 6 is provided with a control unit 63 configured by a control processor and a memory for the operation of the control processor, and a data analyzing unit 64 configured by an analysis processor and a memory for the operation of the analysis processor. The control unit 63 controls the actuation of the device 8, which is configured by a sampler (not shown in the drawing) for automatically supplying sample containers, and a fluid supplying system for preparing and measuring samples, and further performs other actuation controls. The data analyzing unit 64 executes analysis processes such as clustering and the like on each of the distribution data. The result of the analysis is sent to the external data processing unit 3 through an interface 65, and external output and the measurement results are processed, such as external output and storage and the like. The microcomputer 6 is further provided with an interface 66 between the microcomputer 6 and the display and operating unit 7, and an interface 67 between the microcomputer 6 and the device 8.The calculation unit 62 control unit 63, and the interfaces 66 and 67 are connected through a bus 68, and the control unit 63, and the data analyzing unit 64 are connected through a bus 69. The display and operating unit 7 includes a start switch with which the operator starts a measurement, and a touch panel type liquid crystal display unit for displaying the status of the apparatus, the various setting values, and analysis results, as well as receiving input from the operator.

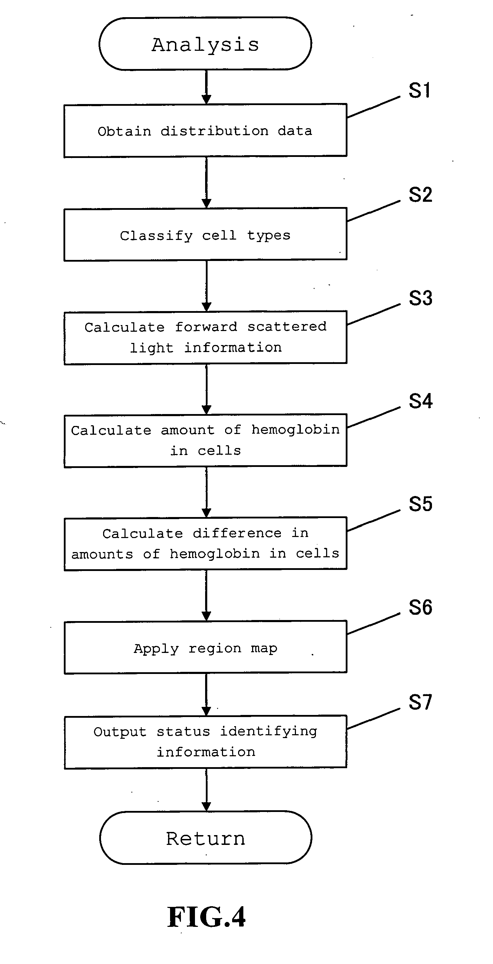

FIG. 4 is a flow chart showing the analysis process performed by the data analyzing unit 64.

First, distribution data of the reticulocyte measurement sample generated by the calculation unit 62 are sent to the data analyzing unit 64 through the buses 63 and 69 (step S1). Clustering is executed on the received distribution data, and each blood cell is classified (step S2).

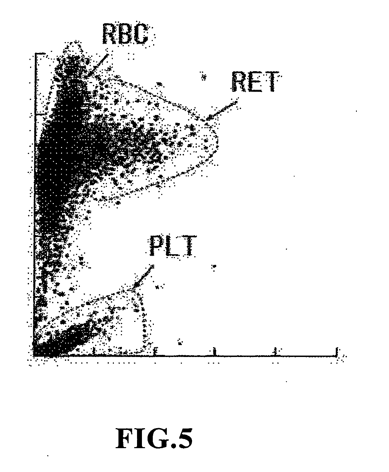

FIG. 5 is a two-dimensional distribution map (scattergram) obtained for the reticulocyte measurement sample. The forward scattered light intensity is on the vertical axis, and the side fluorescent light intensity of on the horizontal axis. The population labeled RBC is the mature red blood cell population, the population labeled RET is the reticulocyte population, and the population labeled PLT is the platelet population. These populations are classified into various types and counted using well known clustering methods. In the present embodiment, the mature red blood cell population RBC and the reticulocyte population RET are respectively identified.

The description now continues with reference to FIG. 4. The average forward scattered light intensity (RBC-Y) of the identified mature red blood cell population is calculated, and the average forward scattered light intensity (RET-Y) of the identified population is calculated (step S3). A value (RBC-He) which reflects the average amount of hemoglobin contained in the mature red blood cells, and a value (RET-He) which reflects the average amount of hemoglobin contained in reticulocytes, and the difference (Delta-He) between these values are calculated from the calculated RBC-Y and RET-Y using a conversion formula. Specifically, these values can be calculated using a dispersion conversion formula.

Equation 1

$$RET-He = A \times exp\ (B \times RET-Y)$$

(Where A=5.8439 and B=0.0098)

Equation 2

$$RBC-He = C \times exp\ (D \times RBC-Y)$$

(Where C=5.8439 and D=0.0098)

Then, the value (RBC-He) is subtracted from the calculated value (RET-He), and the value (Delta-He), which expresses the difference between the two values, is calculated (step S5).

The manner of deriving these equations is described below.

Equation 2 can be derived by measuring a plurality of samples beforehand using a hematology analyze to obtain the forward scattered light intensity (RBC-Y) and MCH (average amount of hemoglobin) of the mature red blood cells for each sample, plotting the analysis results on a graph with the RBC-Y and MCH as axes, and determining the relationship between RBC-Y and MCH. MCH can be conventionally determined by calculating the amount of hemoglobin calculated by the HGB detection unit 10 and the number of red blood cells calculated by the RBC/PLT detection unit 2 shown in FIG. 2. Equation 3 specifically uses a calculation obtained by measuring 500 samples.

Although equation 1 has the same format as equation 2 for simplicity, equation 1 may also be obtained by analyzing a plurality of samples beforehand to obtain the amount of hemoglobin contained in reticulocytes and the average forward scattered light intensity (RET-Y) of reticulocytes, plotting the analysis results in a two-dimensional distribution, and determining the relationship between the RET-Y and the amount of hemoglobin contained in reticulocytes from the condition of the distribution of the two-dimensional distribution. The amount of hemoglobin contained in the reticulocytes may be obtained, for example, using ADVIA 120 (Bayer diagnostics, Inc.).

The methods and means for obtaining values which reflect the amount of hemoglobin contained in reticulocytes and mature red blood cells are not limited to the above descriptions inasmuch as such values may also be obtained by other methods and means. For example, such values may also be obtained by detecting and analyzing two forward scattered lights from different angles relative to the axis, and by detecting and analyzing two forward scattered lights of different wavelengths.

The description now returns to FIG. 4.

Status identifying information (information supporting clinical examination) corresponding to combined RET-He and Delta-He, which has practical use in clinical examinations, is then derived. One such method uses a region map that utilizes a correlation diagram that has RET-He and Delta-He as parameters.

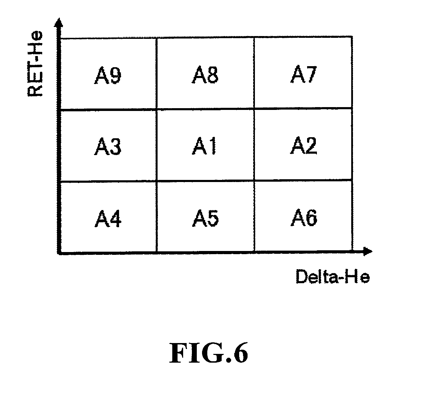

FIG. 6 shows a region map which divides a correlation diagram with RET-He and Delta-He as parameters into a plurality of regions. Region A1is a region in which the RET-He values (28 to 36 pg) and the Delta-He values (0 to 5 pg) are near normal. Region A7 represents a region in which all values are invariably higher than normal, and region A4 represents a region in which all values are lower than normal. Identification codes A1 through A9 appended to each region correspond to the status identifying information (information supporting clinical examination) of each region. The information (the identification code of each region and values stipulating the range of each region) related to the region map is stored in the memory of the data analyzing unit 64 of the microcomputer 6.

The clinical meaning of each region is described below.

Region A1 is a region indicating normal hemoglobin synthesis in normally pigmented red blood cells, region A2 is a region indicating normal hemoglobin synthesis in low pigmented red blood cells, region A3 is a region indicating normal hemoglobin synthesis in high pigmented red blood cells, region A4 is a region indicating potential iron deficiency or a functional iron deficiency condition in reaction to inflammation or acute infection, region A5 is a region indicating iron deficiency or functional iron deficiency condition in reaction to acute infection or inflammation, and region A6 also is a region indicating iron deficiency or functional iron deficiency condition in reaction to chronic infection or inflammation similar to region A5. Region A7 is a region indicating elevated hemoglobin synthesis in normal pigmented red blood cells, region A8 is a region indicating elevated hemoglobin synthesis in high pigmented red blood cells, and region A9 is a region indicating elevated hemoglobin synthesis in extremely high pigmented red blood cells.

Described below is the specific process of adding and outputting information supporting clinical examinations which is practical for clinical use using a previously divided region map in the correlation diagram prepared by combining the two values of RET-He and Delta-He, The process is executed by the data analyzing unit 64.

A determination is made as to whether or not the combination of both values belongs to a region on the region map by supplying a sample to the hematology analyzer and applying the RET-He value and the Delta-He value of the sample calculated by the data analyzing unit 64 to the region map read from the memory of the data analyzing unit 64 (step S6). For example, when the calculated values of Delta-He and RET-He are 2 pg, and 27 pg, respectively, the combination of these values falls lies in region A5, the identifying code A5 which corresponds to region A5 (in this instance, the code name is the same as the region name) is appended to the combination of the values, and RET-He and Delta-He are output with other values from the output unit. In the present embodiment, the values calculated by the data analyzing unit 64 and the corresponding identifying codes are transmitted to the data processing unit 3 through the interface 65, and displayed on the display screen.

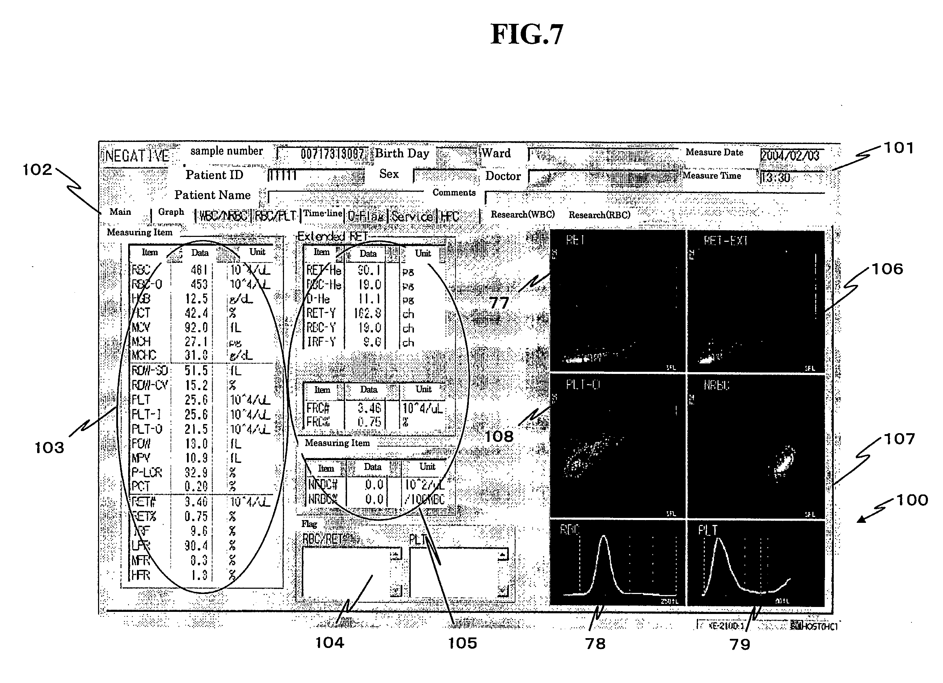

FIG. 7 shows an example of an analysis result screen 100 which is displayed on the display screen of the data processing unit 3. The top part of the screen 100 is provided with an attribute display region 101 for displaying sample or patient attributes, and specifically the sample number, patient name, sex, birth date, ward, attending physician, date of measurement, time of measurement, and comments are displayed. The bottom part of the display screen 101 is provided with a measurement result display region for displaying the results of the measurements. Area 102 displays tabs for switching the content of the display, and has a plurality of tabs for main screen, graph screen, and other items. FIG. 7 shows the condition when the research (RBC) tab has been selected. That is, the analysis results calculated in the present embodiment are held as research items in the hematology analyzer. the left half of the measurement result display region is provided with a text display area for displaying numerical values and flags, and the right half is provided with a distribution map display area for displaying distribution maps. Within the measurement result display area are a display area 103 for displaying the measurement results of measurement items such as RBC, HGB, HCT,...HFR and the like, and a display area 105 for displaying research items such as RET-He, RBC-He, D-He (Delta-He),...NRB% and the like. A flag display area 104 for displaying flags related to red blood cells and platelets is provided below the research display item 105. The identifying codes such as A5, A6, A7 and the like appended to the combined calculation results are displayed in the area 104.

In the present embodiment, six distribution maps are displayed in the distribution map display area; reference number 77 refers to a scattergram of a reticulocyte measurement sample in which the forward scattered light intensity is on the vertical axis and the side fluorescent light intensity is on the horizontal axis, and reference number 106 refers to a scattergram in which the scale of the horizontal axis is changed in the scattergram of 77. Reference number 107 refers to a scattergram of a nucleated red blood cell measurement sample in which the forward scattered light intensity is on the vertical axis and the side fluorescent light intensity is on the horizontal axis, and reference number 108 refers to a scattergram in which the scale of the vertical axis is changed in the scattergram of 77. Reference number 78 refers to a histogram of red blood cells, and reference number 79 refers to a histogram of platelets.

The perspective of using the output identifying codes to monitor treatment is described below. In this case the use of the identifying codes is described in monitoring IDA or ACD, and monitoring EPO or iron supplement treatment. Since iron deficiency anemia expresses small and low pigmented cells, it is usually positioned in region A5. Treatment (medication) begins from this condition. Treatment must be re-evaluated and modified when the values of RET-He and Delta-He calculated after treat has started doe not belong to region A5 or show no effect of treatment. Since a worsening of the anemia is indicated if the values move from region A5 to region A4, the treatment must be re-evaluated. If the values move from region A5 to region A6, the treatment must be continued or modified since partial treatment success is suggested. If the values move from region A6 to region A2, treatment effectiveness is definitely evidenced, and an ultimate move to region A1 can be anticipated.

Monitoring treatment of vitamin B12 or folic acid is described below. Megaloblastic anemia expresses large and high pigmented cells, it usually is positioned in region A7 or A8. The treatment must be regarded as ineffective if the values of RET-He and Delta-He fall in region A 7 or A8 after treatment has been started. If the values move from region A7 or A8 to region A3, the treatment is confirmed to be effective. If this condition continues, the values can be expected to shift to region A1.

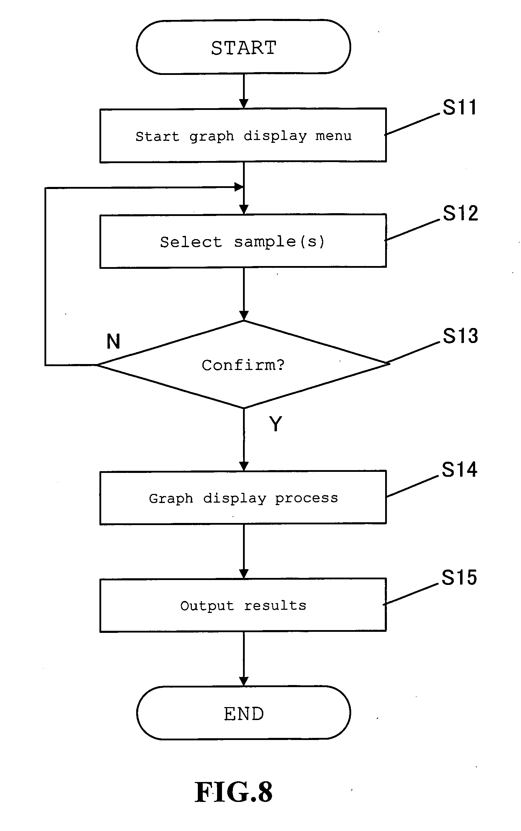

An example of the status identifying information (information supporting clinical examination) output is described below. FIG. 8 is a flow chart. In this example, after obtaining measurement results which include data related to blood hemoglobin from the hematology analyzer, The combined RET-He and Delta-He are plotted on a distribution map of the correlation diagram shown in FIG. 5 to provide status identifying information.

The display screen and keyboard of the data processing unit 3 are first operated to select and start the graph display menu to monitor anemia (step S11). One or more samples on the graph display are selected from a list of stored samples (step S12), and confirmed (step S13). The stored sample is stored on the hard disk of the data processing unit 3, and blood analysis results of each patient as well as patient attribute information are associated and stored. Processing for the graph display of the selected sample is performed by the data processing unit 3 (step S14), and a correlation diagram divided in to region is displayed on the display screen of the data processing unit 3.

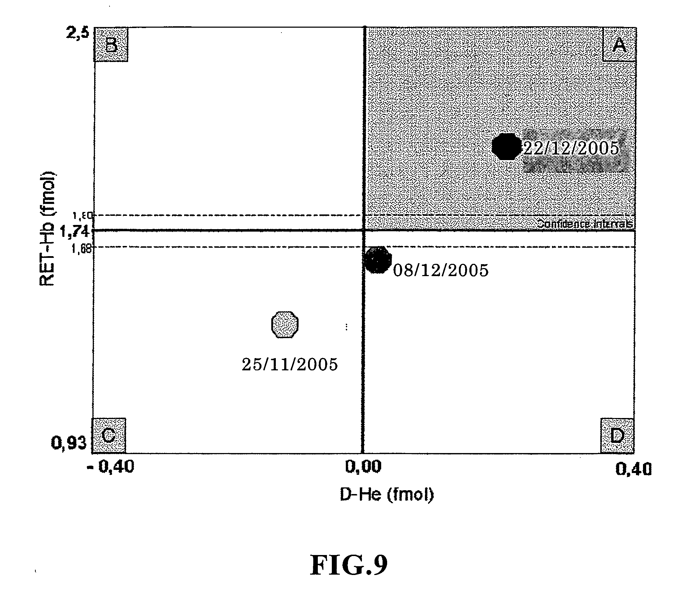

FIG. 9 is a correlation diagram which plots the time coarse of the erythropoietin administration treatment for a patient with iron deficiency anemia. Four regions A, B, C, and D are shown in the display screen. These region are equivalent to regions A1, A3, A4, and A5, respectively. The units are the same as FIG. 6. It is possible to calculate RET-He in fmol units by multiplying the pg units of RET-He by 0.06206. The plot of region C shows RET-He and D-He (Delta-He) data measured on November 25, 2005. The plot in region D on the upper left shows RET-He and D-He data measured on December 8, 2005, and the plot in region A shows RET-He and D-He data measured on December 22, 2005. From the change in values in these plots it can be understood at a glance that the patient improved from a functional iron deficiency condition to a normal condition.

The degree of severity of the anemia and the treatment efficacy can be clearly recognized and grasped visually by outputting a correlation diagram which plots the RET-He and Delta-He data together with the divided regions.

Although red blood cells and reticulocytes are classified using scattered light information and fluorescent light information as optical information in the present embodiment, the present invention is not limited to this arrangement inasmuch as red blood cells and reticulocytes may also be classified, for example, using scattered light information and cell light absorption information.

Although the output of a correlation diagram is described in the above embodiment, the present invention is not limited to this arrangement inasmuch as, for example, a clinical meaning may be output which corresponds to the region in which analysis results are plotted. That is, a summary indicating the patient has normal hemoglobin synthesis in normal pigmented red blood cells may be output when the analysis results are plotted region A1 of the region map shown in FIG. 6; a summary indicating the patient has normal hemoglobin synthesis in low pigmented red blood cells may be output when the analysis results are plotted region A2; a summary indicating the patient has normal hemoglobin synthesis in high pigmented red blood cells may be output when the analysis results are plotted region A3; a summary indicating the patient has potential iron deficiency or functional iron deficiency in reaction to chronic infection or inflammation may be output when the analysis results are plotted region A4; a summary indicating the patient has iron deficiency or functional iron deficiency in reaction to chronic infection or inflammation may be output when the analysis results are plotted region A5 or A6; a summary indicating the patient has high hemoglobin synthesis in normal pigmented red blood cells may be output when the analysis results are plotted region A7; a summary indicating the patient has high hemoglobin synthesis in high pigmented red blood cells may be output when the analysis results are plotted region A8; and a summary indicating the patient has high hemoglobin synthesis in extremely high pigmented red blood cells may be output when the analysis results are plotted region A9.

Although the analysis program for obtaining status identifying information (information supporting clinical examination) is installed beforehand in the data analyzing unit 64 in the above embodiment, the program need not be provided beforehand in the hematology analyzer inasmuch as the analysis program may be provided as a computer program that can realize the functions of the present invention by installing the computer program on a conventional hematology analyzer.

**Claims**

1. A hematology analyzer comprising:

   a detecting unit for irradiating a blood sample with light and obtaining optical information which comprises at least scattered light information from each cell contained in the blood sample;
   a cell classifying means for classifying red blood cells and reticulocytes among the cells based on the obtained optical information;
   a scattered light information obtaining means for obtaining red blood cell scattered light information which is scattered light information related to red blood cells, and reticulocyte scattered light information which is scattered light information related to reticulocytes based on the classification results of the cell classifying means;
   a hemoglobin amount obtaining means for obtaining a value equivalent to the amount of hemoglobin in the red blood cells from the red blood cell scattered light information, a value equivalent to the amount of hemoglobin in the reticulocytes from the reticulocyte scattered light information, and difference between the hemoglobin amounts which is the difference between the value equivalent to the amount of hemoglobin in the red blood cells and the value equivalent to the amount of hemoglobin in the reticulocytes; and

a supporting information obtaining means for obtaining information supporting clinical examination based on the difference between the hemoglobin amounts and the value equivalent to the amount of hemoglobin in reticulocytes.

2. The hematology analyzer of claim 1, further comprising an output means for outputting the value equivalent to the amount of hemoglobin in reticulocytes, the difference between the hemoglobin amounts, and the information supporting clinical examination.

3. The hematology analyzer of claim 1, further comprising a memory for storing the information supporting clinical examination related to the value equivalent to the amount of hemoglobin in reticulocytes and the difference between the hemoglobin amounts; wherein the supporting information obtaining means reads the corresponding information supporting clinical examination from the memory based on the difference between the hemoglobin amounts and the values equivalent to the amount of hemoglobin in reticulocytes obtained by analyzing a blood sample.

4. The hematology analyzer of claim 2, further comprising a memory for storing correlation diagrams divided into a plurality of regions corresponding to the information supporting clinical examination; wherein the supporting information obtaining means obtains the information supporting clinical examination by plotting, on the correlation diagram read from the memory, marks, as sample analysis information, representing the difference between the hemoglobin amounts and the values equivalent to the amount of hemoglobin in reticulocytes obtained by analyzing a blood sample, and the output means outputs the correlation diagram containing the plotted sample analysis information as the information supporting clinical examination.

5. The hematology analyzer of claim 4, further comprising a second memory for storing sample attributes and the correlation diagrams containing plotted sample analysis information; wherein the supporting information obtaining means plots marks representing the difference between the hemoglobin amounts and the values equivalent to the amount of hemoglobin in reticulocytes as a second sample analysis information on the correlation diagram read from the second memory when a blood sample is analyzed which has sample attribute information that is identical to the sample attribute information of the correlation diagram stored in the second memory.

6. The hematology analyzer of claim 5, wherein the plotted sample analysis information, and analysis date are displayed in the correlation diagram.

7. The hematology analyzer of any one among claims 1 through 6, wherein the red blood cell scattered light information is an average value of the scattered light intensity of the red blood cells, and the reticulocyte scattered light information is an average value of the scattered light intensity of the reticulocytes.

8. The hematology analyzer of any one among claims 1 through 7, wherein the information supporting clinical examination comprises information representing the status of hemoglobin synthesis.

9. The hematology analyzer of any one among claims 1 through 8, wherein the information supporting clinical examination comprises information representing the status of iron deficiency.

10. The hematology analyzer of any one among claims 1 through 9, wherein the information supporting clinical examination is information which is used to comprehend the efficacy of anemia treatment.

11. A hematology analyzing method, comprising steps of:

obtaining red blood cell scattered light information which is scattered light information related to red blood cells contained in a blood sample, and reticulocyte scattered light information which is scattered light information related to reticulocytes contained in the blood sample;
obtaining a value equivalent to the amount of hemoglobin in the red blood cells from the red blood cell scattered light information;
obtaining a value equivalent to the amount of hemoglobin in the reticulocytes from the reticulocyte scattered light information;
obtaining difference between the hemoglobin amounts which is the difference between the value equivalent to the amount of hemoglobin in the red blood cells and the value equivalent to the amount of hemoglobin in the reticulocytes; and
obtaining information supporting clinical examination based on the value equivalent to the amount of hemoglobin

EP 1 967 840 A2

in the reticulocytes and the difference between the hemoglobin amounts.

12. The hematology analyzing method of claim 11, further comprising a step of irradiating a blood sample with light and obtaining optical information which includes at least scattered light information from each cell contained in the blood sample; and

a step of classifying red blood cells and reticulocytes among the cells based on the obtained optical information; wherein the red blood cell scattered light information and the reticulocyte scattered light information are obtained based on the result of classifying the red blood cells and reticulocytes.

13. The hematology analyzing method of claim 11, further comprising a step outputting the value equivalent to the amount of hemoglobin in the reticulocytes, the difference between the hemoglobin amounts, and the information supporting clinical examination.

14. The hematology analyzing method of claim 13 further comprising a step of storing in a memory correlation diagrams which are divided into a plurality of regions corresponding to the information supporting clinical examination using the value equivalent to the amount of hemoglobin in the reticulocytes and the difference between the hemoglobin amounts as parameters;
wherein the step of obtaining supporting information comprises a step of obtaining the information supporting clinical examination by plotting, on the correlation diagram read from the memory, marks, as sample analysis information, representing the difference between the hemoglobin amounts and the values equivalent to the amount of hemoglobin in reticulocytes obtained by analyzing a blood sample; and the output step comprises a step of outputting the correlation diagram containing the plotted sample analysis information as the information supporting clinical examination.

15. The hematology analyzing method of claim 11, further comprising a step of storing in a memory the information for supporting clinical examination corresponding to the value equivalent to the amount of hemoglobin in the reticulocytes and the difference between the hemoglobin amounts;
wherein the step of obtaining supporting information further comprises a step of reading the corresponding information supporting clinical examination from the memory based on the difference between the hemoglobin amounts and the values equivalent to the amount of hemoglobin in reticulocytes obtained by analyzing a blood sample.

16. A computer program product, comprising:

a computer readable medium; and
instructions, on the computer readable medium, adapted to enable a general purpose computer to perform operations, comprising:
a step of obtaining red blood cell scattered light information which is scattered light information related to red blood cells contained in a blood sample, and reticulocyte scattered light information which is scattered light information related to reticulocytes contained in the blood sample;
a step of obtaining a value equivalent to the amount of hemoglobin in the red blood cells from the red blood cell scattered light information;
a step of obtaining a value equivalent to the amount of hemoglobin in the reticulocytes from the reticulocyte scattered light information;
a step of obtaining difference between the hemoglobin amounts which is the difference between the value equivalent to the amount of hemoglobin in the red blood cells and the value equivalent to the amount of hemoglobin in the reticulocytes; and
a step of obtaining information supporting clinical examination based on the value equivalent to the amount of hemoglobin in the reticulocytes, and the difference between the hemoglobin amounts.

17. The computer program product of claim 16
wherein the instructions further comprise:

a step of irradiating a blood sample with light and obtaining optical information which comprises at least scattered light information from each cell contained in the blood sample; and
a step of classifying red blood cells and reticulocytes among the cells based on the obtained optical information; wherein the red blood cell scattered light information and the reticulocyte scattered light information are obtained based on the result of classifying the red blood cells and reticulocytes.

**18.** The computer program product of claim 16
wherein the instructions further comprise:

a step of outputting the value equivalent to the amount of hemoglobin in the reticulocytes, the difference between the hemoglobin amounts, and the information supporting clinical examination.

**19.** The computer program product of claim 18
wherein the instructions further comprise:

a step of storing in a memory correlation diagrams which are divided into a plurality of regions corresponding to the information supporting clinical examination using the value equivalent to the amount of hemoglobin in the reticulocytes and the difference between the hemoglobin amounts as parameters;
wherein the step of obtaining supporting information comprises a step of obtaining information supporting clinical examination by plotting on the correlation diagram read from the memory, marks, as sample analysis information, representing the difference between the hemoglobin amounts and the values equivalent to the amount of hemoglobin in reticulocytes obtained by analyzing a blood sample; and
the output means outputs the correlation diagram containing the plotted sample analysis information as the information supporting clinical examination.

**20.** The computer program product of claim 16
wherein the instructions further comprise:

a step of storing in a memory the information supporting clinical examination corresponding to the value equivalent to the amount of hemoglobin in the reticulocytes and the difference between the hemoglobin amounts;
wherein the step of obtaining supporting information further comprises a step of reading the corresponding information supporting clinical examination from the memory based on the difference between the hemoglobin amounts and the values equivalent to the amount of hemoglobin in reticulocytes obtained by analyzing a blood sample.

**FIG.1**

FIG.2

**FIG.3**

```
          ┌─────────────────────┐
          │      Analysis       │
          └─────────────────────┘
                     │
                     ▼
          ┌─────────────────────┐         S1
          │ Obtain distribution │
          │        data         │
          └─────────────────────┘
                     │
                     ▼
          ┌─────────────────────┐         S2
          │  Classify cell types│
          └─────────────────────┘
                     │
                     ▼
          ┌─────────────────────┐         S3
          │ Calculate forward   │
          │ scattered light     │
          │ information         │
          └─────────────────────┘
                     │
                     ▼
          ┌─────────────────────┐         S4
          │ Calculate amount of │
          │ hemoglobin in cells │
          └─────────────────────┘
                     │
                     ▼
          ┌─────────────────────┐         S5
          │ Calculate difference│
          │ in amounts of       │
          │ hemoglobin in cells │
          └─────────────────────┘
                     │
                     ▼
          ┌─────────────────────┐         S6
          │  Apply region map   │
          └─────────────────────┘
                     │
                     ▼
          ┌─────────────────────┐         S7
          │ Output status       │
          │ identifying         │
          │ information         │
          └─────────────────────┘
                     │
                     ▼
          ┌─────────────────────┐
          │      Return         │
          └─────────────────────┘
```

**FIG.4**

14

**FIG.5**

**FIG.6**

# FIG.7

EP 1 967 840 A2

NEGATIVE   sample number [ 007173]3097] Birth Day [        ] Ward [        ]        Measure Date [2004/02/03]  ⟋101

Patient ID [11111]        Sex [        ]  Doctor [        ]  Measure Time [13:30]

Patient Name [        ]        Comments [        ]

Main | Graph | WBC/NRBC | RBC/PLT | Time-line | Q-Flag | Service | HPC | Research(WBC)  Research(RBC)

Measuring Item        Extended RET

| Item | Data | Unit |
|------|------|------|
| RBC | 461 | 10^4/uL |
| RBC-O | 453 | 10^4/uL |
| HGB | 12.5 | g/dL |
| HCT | 42.4 | % |
| MCV | 92.0 | fL |
| MCH | 27.1 | pg |
| MCHC | 31.0 | g/dL |
| RDW-SD | 51.5 | fL |
| RDW-CV | 15.2 | % |
| PLT | 25.6 | 10^4/uL |
| PLT-I | 25.6 | 10^4/uL |
| PLT-O | 21.5 | 10^4/uL |
| PDW | 13.0 | fL |
| MPV | 10.9 | fL |
| P-LCR | 32.9 | % |
| PCT | 0.20 | % |
| RET# | 3.46 | 10^4/uL |
| RET% | 0.75 | % |
| IRF | 9.6 | % |
| LFR | 90.4 | % |
| MFR | 8.3 | % |
| HFR | 1.3 | % |

| Item | Data | Unit |
|------|------|------|
| RET-He | 90.1 | pg |
| RBC-He | 19.0 | pg |
| D-He | 11.1 | pg |
| RET-Y | 162.8 | ch |
| RBC-Y | 19.0 | ch |
| IRF-Y | 9.6 | ch |

| Item | Data | Unit |
|------|------|------|
| FRC# | 3.46 | 10^4/uL |
| FRC% | 0.75 | % |

Measuring Item

| Item | Data | Unit |
|------|------|------|
| NRBC# | 0.0 | 10^2/uL |
| NRBC% | 0.0 | /100RBC |

Flag
RBC/RET        PLT

RET        RET-EXT

PLT-O        NRBC

RBC        PLT

XE-2100-1    HOST(HC1)

102  103  104  105  77  108  78  79  106  107  100

START

Start graph display menu — S11

Select sample(s) — S12

Confirm? — S13
N
Y

Graph display process — S14

Output results — S15

END

**FIG.8**

**FIG.9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005219527 A
- JP 2005257450 A